# EUROPEAN PATENT APPLICATION

(11) **EP 1 369 488 A1**
(43) Date of publication of application: **10.12.2003**
(21) Application number: 02012699.1
(22) Date of filing: 07.06.2002
(51) Int. Cl.: C12P 7/18, C12N 1/38, C12R 1/22, C12R 1/24, C12R 1/145, C12R 1/185, C12R 1/01

(54) **Medium for the fermentive production of 1,3-propanediol, process and microorganism**

(71) Applicant: Gesellschaft für Biotechnologische Forschung mbH (GBF), 38124 Braunschweig (DE)
(72) Inventor: Zeng, An-Ping, 38124 Braunschweig (DE); Hartlep, Michael, 38124 Braunschweig (DE)
(74) Representative: Boeters, Hans Dietrich, Dr.

(57) **Abstract**

This invention deals with a new medium formulation and a fed-batch process for the microbial production of 1,3-propanediol. The compound 1,3-propanediol is of economical importance. It can be used as a monomer for the synthesis of cyclic compounds.

## Description

This invention deals with a new medium formulation and a fed-batch process for the microbial production of 1,3-propanediol. The compound 1,3-propanediol is of economical importance. It can be used as a monomer for the synthesis of polyesters, polyethers and polyurethanes and as an intermediate for the synthesis of cyclic compounds.

### State-of-the-art of the technology

There exist several chemical and microbial processes for the production of 1,3-propanediol. Chemically, 1,3-propanediol can be produced either from acrolein or from ethylene oxide. Acrolein is hydrated at moderate temperature and pressure to 3-hydroxypropionaldehyde which, in a second reaction, is hydrogenated to 1,3-PD at a rubidium catalyst under high pressure (Sullivan C.J. 1993, Ullmann's Encyclopedia of Industrial Chemistry Vol. A22: 163-171). Ethylene oxide is transformed with synthesis gas in a hydroformulation process to 3-hydroxypropanal as well, but for this reaction very high pressure (150 bar) is required. The aldehyde is extracted from the organic phase with water and subjected to hydrogenation using nickel as a catalyst, again under high pressure. Biotechnologically, 1,3-propanediol can be produced from glycerin by native organisms or from glucose by genetically recombinant organisms. A number of strains from the genera *Lactobacillus (L. brevis), Enterobacter (E. agglomerans), Citrobacter (C. freundii), Klebsiella (K. pneumoniae)* und *Clostridium (C. butyricum)* are known to be able to convert glycerol to 1,3-propanediol (Biebl H. et al. 1999, Appl. Microbiol. Biotechnol. 52: 289-297). *Echerichia coli* and *Saccharomyces cerivesiae* were studied to convert glucose to 1,3-propanediol.

The till now published highest concentration of 1,3-propanediol produced from glycerol was 73.3 g/L (Cameron D.C. et al. 1998, Biotechnol. Prog. 14: 116-125). This concentration was achieved in a fed-batch process with *K. pneumoniae* under the use of over 20 g/L yeast extract in the culture medium. Table 1 gives a summary of results reported for the microbial production of 1,3-propanediol from glycerol.

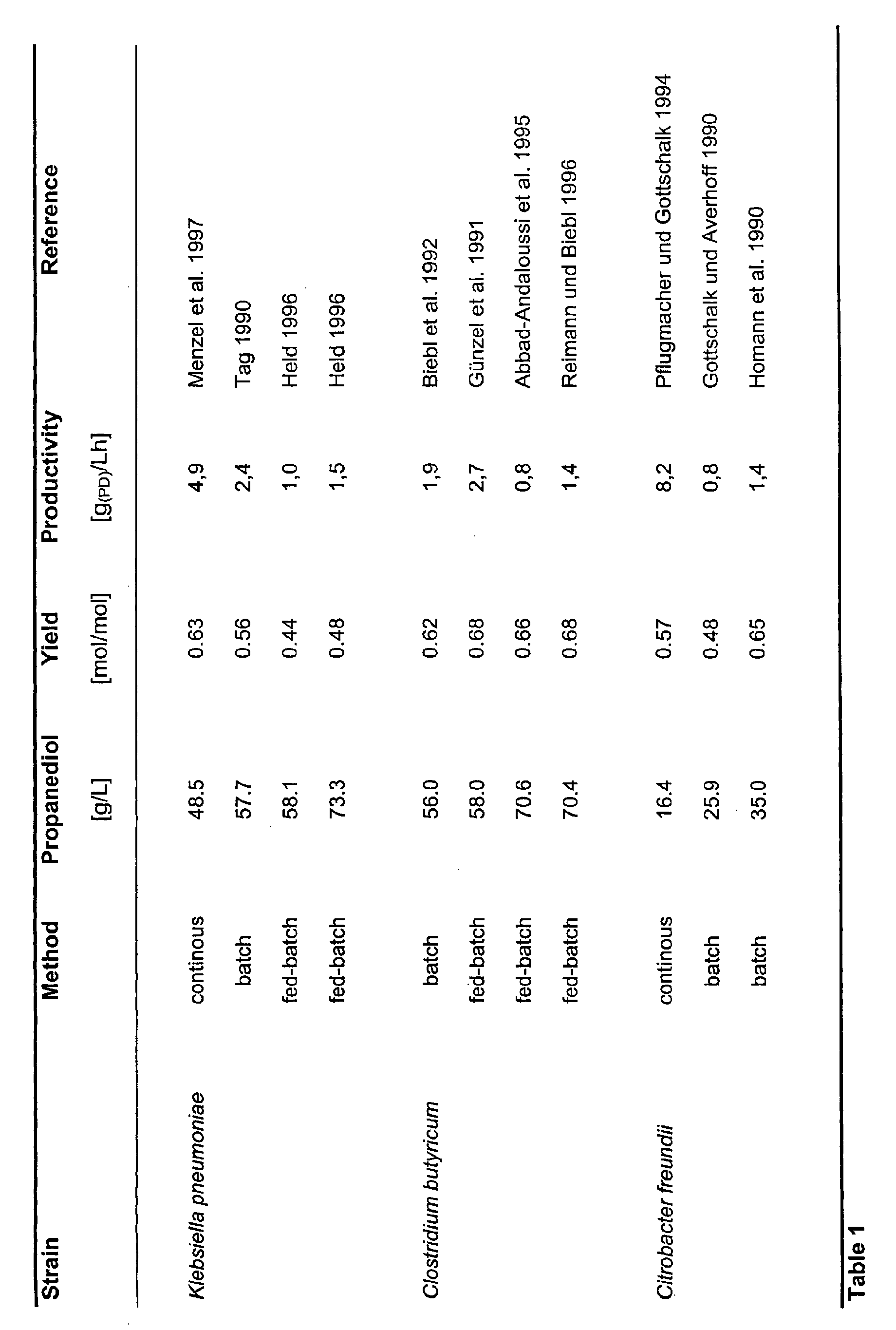

According to one embodiment the problem underlaying the invention is solved by a medium for a fermentative production of 1,3-propanediol from glycerol, wherein the medium contains an organic sulfur compound.

The medium according to the invention may contain L-cysteine, L-cystine, glutathione and/or methionine as organic sulfur compound.

Further, the medium according to the invention may be based on a known medium for a fermentative production of 1,3-propanediol from glycerol, especially on the Evans medium as known medium.

Further, the medium according to the invention may be based on a medium for a fermentation of
- Klebsiella, especially Klebsiella pneumoniae,
- Clostridium, especially Clostridium butyricum,
- Citrobacter, especially Citrobacter freundii,
- Lactobacillus, especially Lactobacillus brevis,
- Enterobacter, especially Enterobacter agglomerans, or
- Escherichia, especially recombinant Escherichia, preferably Escherichia coli, especially recombinant Escherichia coli.

Further, the medium according to the invention may have a content of 0.01 to 2.0 g organic sulfur compound/kg and especially 0.01 to 1.0 g organic sulfur compound/kg, such as a content of 0.05 to 2 g L-cysteine/kg with L-cysteine as organic sulfur compound.

Further, the medium according to the invention may have a content of 500 to 850 g glycerol/kg.

Further, the medium according to the invention may have a content of 0.5 to 8.5 and especially 1.0 to 5.0 g yeast extract/kg.

Further, the medium according to the invention may contain NH₄Cl, KCl, NaH₂PO₄, Na₂SO₄, MgCl₂, CaCl₂ and/or citric acid.

Further, the medium according to the invention may contain FeCl₃, MnCl₂, ZnCl₂, COCl₂, CuCl₂, H₃BO₃, Na₂MoO₄ and/or HCl as trace elements.

Finally, the medium according to the invention may contain an anti-foaming agent.

According to another embodiment the problem underlaying the invention is solved by a process for a fermentative production of 1,3-propanediol from glycerol by means of a medium according to the invention.

The process according to the invention may be carried out as batch fermentation or as fed-batch fermentation.

According to another embodiment the problem underlaying the invention is solved by a microorganism for use in a process according to the invention, wherein the microorganism converts fermentatively glycerol to 1,3-propanediol and wherein the microorganism can be obtained by a process which starts from available microorganism strains and subjects these microorganism strains to a screening and selects a microorganism strain which overexpresses cysteine synthase.

The microorganism according to the invention can be obtained by a process which starts from microorganism strains which are selected from the group of Klebsiella, Clostridium, Citrobacter, Lactobacillus, Enterobacter and Escherichia, especially recombinant Escherichia, wherein the process may start from microorganism strains selected from the group of Klebsiella pneumoniae, Clostridium butyricum, Citrobacter freundii, Lactobacillus brevis, Enterobacter agglomerans and Escherichia coli, especially recombinant Escherichia coli.

Further, the process may start from deposited microorganism strains.

Finally, the process may start from microorganism strains isolated from natural sources.

An explanation of the invention by 4 examples of media and by figures follows, which show:
Fig. 1 Comparison of biomass formation in fed-batch fermentations of glycerol by *K. pneumoniae* with Evans-medium and the invented medium.
Fig. 2 Comparison of 1,3-propanediol formation in fed-batch fermentations of glycerol by *K. pneumoniae* with Evans-medium and the invented medium.
Fig. 3 Comparison of 1,3-propanediol formation in fed-batch fermentations of glycerol by *C. butyricum* with Medium 3 and the invented medium.

### Media

**Medium 1:** Medium for preculture and shake-flask cultures
(According to Homann et al. 1990, Appl. Microbiol. Biotechnol. 33: 121 - 126)

| | | | |
|---|---|---|---|
| Perculture medium | Glycerol | 20,0 | g/L |
| | K₂HPO₄ | 3,4 | g/L |
| | KH₂PO₄ | 1,3 | g/L |
| | (NN₄)₂SO₄ | 2,0 | g/L |
| | CaCO₃ | 2,0 | g/L |
| | Yeast extract (DIFCO) | 1,0 | g/L |
| | MgS0₄· 7 H₂O | 0,2 | g/L |
| | CaCl₂· H₂O | 0,02 | g/L |
| | | | |
| | Iron surfate solution | 1,0 | mL/L |
| | Trace element solution A | 2,0 | mL/L |

| | | | |
|---|---|---|---|
| Iron surfate solution: | FeSO₄ · 7 H₂O | 5,0 | g/L |
| | HCI (concentrated) | 4,0 | mL/L |
| Trace element solution A: | CoCl₂ · 6 H₂O | 200 | mg/L |
| | MnCl₂ · 4 H₂O | 100 | mg/L |
| | ZnCl₂ | 70 | mg/L |
| | H₃BO₃ | 60 | mg/L |
| | Na₂MoO₄ · 2 H₂O | 35 | mg/L |
| | NiCl₂ · 6 H₂O | 25 | mg/L |
| | CuCl₂ · 2 H₂O | 20 | mg/L |
| | HCI (concentrated) | 0,9 | mL/L |

**Medium 2:** Fermentation medium
(According to Evans et al. 1970, Methods in Microbiology Vol.2: 277 - 327)

| | | | |
|---|---|---|---|
| Fermentation medium | NH₄Cl | 5,35 | g/L |
| | KCI | 0,75 | g/L |
| | NaH₂PO₄ · H₂O | 1,38 | g/L |
| | Na₂SO₄ | 0,28 | g/L |
| | MgCl₂ · 6 H₂O | 0,26 | g/L |
| | Citric acid · H₂O | 0,42 | g/L |
| | CaCl₂ · H₂O | 2,90 | mg/L |
| | Yeast extract (DIFCO) | 1,00 | g/L |
| | Glycerol | | varied |
| | Trace element solution B | 5,0 | mL/L |
| | Desmophen | 0,1 | mL/L |

| | | | |
|---|---|---|---|
| Trace element solution B | FeCl₃ · 6 H₂O | 5,4 | g/L |
| | MnCl₂ · 4 H₂O | 2,0 | g/L |
| | ZnCl₂ | 684 | mg/L |
| | CoCl₂ · 6 H₂O | 476 | mg/L |
| | CuCl2 · 2 H₂O | 170 | mg/L |
| | H₃BO₃ | 62 | mg/L |
| | Na₂MoO₄ · 2 H₂O | 5 | mg/L |
| | HCI (concentrated) | 10,0 | mL/L |

**Medium 3:** Homann's Medium for Clostridia (Homann et al. 1990, Appl. Microbiol. Biotechnol. 33: 121 - 126; Abbad-Andaloussi et al. 1995, Appl. Environ. Microbiol. 61, 4413-4417) with following changes:

| | |
|---|---|
| K₂HPO₄ | 1.0 g/L and |
| KH₂PO₄ | 0.5 g/L |

**Medium 4.** the invented new medium. This medium is based on modifications of the above mentioned three media (Medium 1-3). In particular, organic sulphur compounds are added to the medium which can result in a significant improvement of 1,3-production with different organisms.

### Example 1

### Growth of K. pneumoniae in anaerobic flasks

The experiments were done in 100 mL anaerobic flasks with screw cap and septum. The flasks were filled with 50 mL Homan-medium (Medium 1) under gassing with nitrogen and then autoclaved. 5 mL solution (pH=7.0) was added to each of the flasks after autoclaving. In the reference flasks 5 mL distilated water was added, while 5 mL of a solution containing 0.01-2 g/L L-cystein was added to other flasks. These flasks were inoculated with 5 mL *K.pneumoniae* from an aerobic preculture and incubated at 37°C for 24 h in an incubator.

### Results:

The average optic density (650 nm) (OD) in the reference flasks increased from 0.31 to about 0.94, corresponding to an OD difference of 0.63. In the flasks containing L-cystein solution OD was increased from 0.26 to 1.06, corresponding to an OD difference of 0.80. The biomass formation in these flasks was thus 127% of that in the reference flasks.
The average consumption of glycerol in the flasks containing cystein was 148 mM. This corresponds to an increase by 28% compared to the consumption of glycerol (116 mM) in the reference flasks.

### Example 2

### Growth of K. pneumoniae in anaerobic flasks containing glutathione

The experiments were done in 100 mL anaerobic flasks with screw cap and septum. The flasks were filled with 50 mL Homan-medium (Medium 1) under gassing with nitrogen and then autoclaved.

5 mL solution (pH=7.0) was added to each of the flasks after autoclaving. In the reference flasks 5 mL distilated water was added, while 5 mL of a glutathione containing solution was added to other flasks. These flasks were inoculated with 5 mL *K.pneumoniae* from an aerobic preculture and incubated at 37°C for 24 h in an incubator.

### Results:

The average optic density (650 nm) (OD) in the reference flasks increased from 0.31 to about 0.94, corresponding to an OD difference of 0.63. In the flasks containing gluthathione solution the average OD was increased from 0.25 to 1.03, corresponding to an OD difference of 0.78. The biomass formation in these flasks was thus 124% of that in the reference flasks.
The average consumption of glycerol in the flasks containing gluthathione was 141 mM. This corresponds to an increase by 22% compared to the consumption of glycerol (116 mM) in the reference flasks.

### Example 3

### Growth and 1,3-propanediol formation in fed-batch fermentation of glycerol by K. pneumoniae

Fed-batch fermentations were carried out in a 4 1 Setric Bioreactor (Set 4V, Setric Genic Industriel, Toulouse, France) with a starting volume of 2.2 1. The bioreactor was connected to a real-time computer control system for on-line data acquisition and control of medium feeding. The cultivation conditions were as follows: Evans-medium or the invented medium, temperature 37°C, pH 7.0, and 150 rpm for agitation. To ensure anaerobic conditions, the bioreactor was sparged with nitrogen at a flow rate of 0.4 volume per volume per minute.

The normal feeding solution contained 85 % glycerol and 0.5 % yeast extract. The improved feeding solution contained 85 % glycerol, 0.5 % yeast extract and added sulfur compounds in a concentration range of 0.01 -1 g/L. The medium feeding was started after consumption of 10 g alkaline solution (20% NaOH), normally about 4 hours after inoculation. The feeding rate was coupled with the alkaline consumption rate.

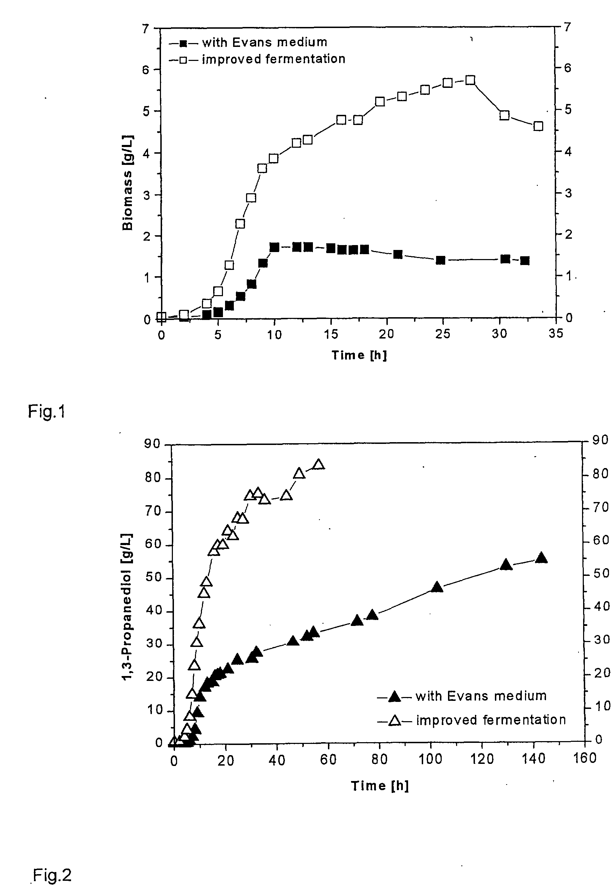

The use of the invented medium and the improved feeding strategy led to a significant increase in both cell growth and 1,3-propanediol formation compared to normal fed-batch fermentations with the afore mentioned Evans-medium as shown in Figs. 1 and 2. A final 1,3-propanediol concentration as high as 83.5 g/L was achieved in a much shorter fermentation time with the invented medium. The productivity was increased by about two-folds.

### Example 4

### 1,3-propanediol formation in fed-batch fermentation of glycerol by Clostridium butyricum

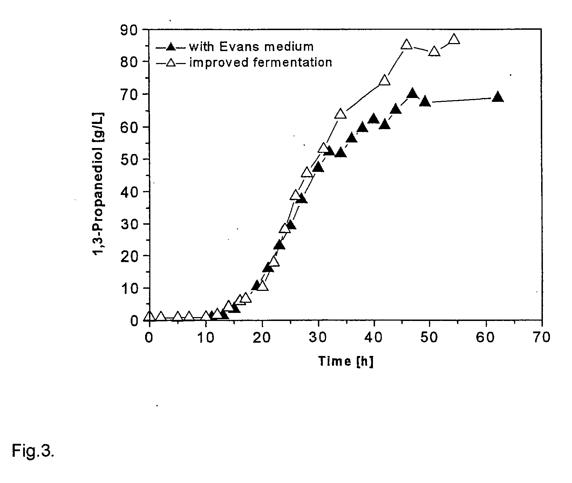

The conditions for fed-batch fermentations of glycerol with *Clostridium butyricum* were similar as in Example 3 except that the modified Homann-medium (Medium 3) was used as the basal medium and the aeration rate was reduced to 0.1 vvm. With the invented medium a biomass concentration of 4.8 g/L and a 1,3-propanediol concentration of 86.6 g/L were achieved, compared to about 68.8 g/L 1,3-propanediol in a comparative fed-batch fermentation with Medium 3 (Fig.3).

## Claims

1. Medium for a fermentative production of 1,3-propanediol from glycerol, wherein the medium contains an organic sulfur compound.

2. Medium according to claim 1, wherein the medium contains L-cysteine, L-cystine, glutathione and/or methionine as organic sulfur compound.

3. Medium according to claim 1 and/or claim 2 based on a known medium for a fermentative production of 1,3-propanediol from glycerol.

4. Medium according to claim 3 based on the Evans medium as known medium.

5. Medium according to claim 1 and/or claim 2 based on a medium for a fermentation of
- Klebsiella, especially Klebsiella pneumoniae,
- Clostridium, especially Clostridium butyricum,
- Citrobacter, especially Citrobacter freundii,
- Lactobacillus, especially Lactobacillus brevis,
- Enterobacter, especially Enterobacter agglomerans, or
- Escherichia, especially recombinant Escherichia, preferably Escherichia coli, especially recombinant Escherichia coli.

6. Medium according to any of the preceding claims with a content of 0.01 to 2.0 g organic sulfur compound/kg and especially 0.01 to 1.0 g organic sulfur compound/kg.

7. Medium according to claim 6 with a content of 0.05 to 2 g L-cysteine/kg with L-cysteine as organic sulfur compound.

8. Medium according to any of the preceding claims with a content of 500 to 850 g glycerol/kg.

9. Medium according to any of the preceding claims with a content of 0.5 to 8.5 and especially 1.0 to 5.0 g yeast extract/kg.

10. Medium according to any of the preceding claims, wherein the medium contains NH₄Cl, KCl, NaH₂PO₄, Na₂SO₄, MgCl₂, CaCl₂ and/or citric acid.

11. Medium according to any of the preceding claims, wherein the medium contains FeCl₃, MnCl₂, ZnCl₂, CoCl₂, CuCl₂, H₃BO₃, Na₂MoO₄ and/or HCl as trace elements.

12. Medium according to any of the preceding claims, wherein the medium contains an anti-foaming agent.

13. Process for a fermentative production of 1,3-propanediol from glycerol by means of a medium according to any of the preceding claims.

14. Process according to claim 13, wherein the process is carried out as batch fermentation or as fed-batch fermentation.

15. Microorganism for use in a process according to claim 13 and/or claim 14, wherein the microorganism converts fermentatively glycerol to 1,3-propanediol and wherein the microorganism can be obtained by a process which starts from available microorganism strains and subjects these microorganism strains to a screening and selects a microorganism strain which overexpresses cysteine synthase.

16. Microorganism according to claim 15, wherein the process starts from microorganism strains which are selected from the group of Klebsiella, Clostridium, Citrobacter, Lactobacillus, Enterobacter and Escherichia, especially recombinant Escherichia.

17. Microorganism according to claim 16, wherein the process starts from microorganism strains selected from the group of Klebsiella pneumoniae, Clostridium butyricum, Citrobacter freundii, Lactobacillus brevis, Enterobacter agglomerans and Escherichia coli, especially recombinant Escherichia coli.

18. Microorganism according to any of claims 15 to 17, wherein the process starts from deposited microorganism strains.

19. Microorganism according to any of claims 15 to 17, wherein the process starts from microorganism strains isolated from natural sources.
